# EUROPÄISCHE PATENTANMELDUNG

(11) **EP 4 674 362 A1**
(43) Veröffentlichungstag der Anmeldung: **07.01.2026**
(21) Anmeldenummer: 24186041.0
(22) Anmeldetag: 02.07.2024
(51) Int. Cl.: A61B 17/295, A61B 18/12, A61B 17/20, A61B 18/14, A61B 17/29, A61B 18/00, A61B 34/30

(54) **WERKZEUGEINHEIT FÜR EIN KOAGULATIONS- UND DISSEKTIONSINSTRUMENT**

(71) Anmelder: Erbe Elektromedizin GmbH, 72072 Tübingen (DE)
(72) Erfinder: LOESER, David, 72108 Rottenburg (DE); PAESCH, Markus, 72461 Albstadt (DE); BOTT, Hanah, 72074 Tuebingen (DE); SCHAEFER, Christian, 72127 Kusterdingen (DE)
(74) Vertreter: Rüger Abel Patentanwälte PartGmbB

(57) **Zusammenfassung**

Eine erfindungsgemäße Werkzeugeinheit (15) weist zwei gegeneinander elektrisch isolierte oder aus isolierendem Material bestehende Branchen (25, 27) auf, an denen Koagulationselektroden (26, 28) vorgesehen sind. Während die erste Branche (25) an der Werkzeugeinheit starr gehalten ist, ist die zweite Branche (27) schwenkbar. Um der zweiten Branche (27) eine Schwenkbewegung zu erteilen, ist ein Betätigungselement (30) in Form einer Zugdruckstange, beispielsweise in Gestalt eines flexiblen Blechstreifens vorgesehen, der zugleich als elektrischer Leiter dient, um der zweiten Koagulationselektrode (28) Strom zuzuführen. Die erste Koagulationselektrode (26) ist über ein Anschlussmittel mit einer Zuleitungslitze (21) verbunden. Das Anschlussmittel kann die Branche (25) selbst, ein in dieser vorgesehenes elektrisch leitendes Inlay oder einer Anschlussleitung sein. Eine zweite Anschlussleitung (34) dient zur Kontaktierung der Schneidelektrode (33), das heißt zur Stromzuführung zu derselben.

## Beschreibung

Die Erfindung betrifft eine Werkzeugeinheit für ein Koagulations- und Dissektionsinstrument.

Koagulations- und Dissektionsinstrumente dienen typischerweise dazu, biologisches Gewebe zwischen beweglichen Branchen eines Werkzeugteils zu fassen und durch Bestromung zu koagulieren und gegebenenfalls auch zu durchtrennen. Typische Beispiele solcher Instrumente sind aus der DE 20 2007 009 165 U1, der EP 3 632 354 A1, der DE 10 2004 013 530 A1, der US 6 656 177 B2, der US 2005/0113826 A1, der US 2002/0115997 A1, der US 8,394,094 B2, der US 6,113,598 B2, der US 2011/0082494 A2, der US 2013/0085516 A1, der DE 10 2006 062 848 B4, der US 6 585 735 B1, der US 2002/0143358 A1, der US 2003/0216733 A1, der WO 2008/040483 A1, der US 2004/0044363 A1 und der EP 2 845 548 B1 entnehmbar. Diese Instrumente haben jeweils einen länglichen Schaft, an dessen distalem Ende zwei Branchen gehalten sind, von denen wenigstens eine beweglich gelagert ist, um mit der anderen Branche zusammen ein zangenähnliches Maulteil zu bilden. Die Branchen sind zugleich Elektroden oder mit Elektroden versehen. Zu dem Maulteil kann außerdem ein beweglich gelagertes Messer oder auch eine Schneidelektrode gehören. Die Elektroden dienen zur Bestromung des zwischen ihnen gefassten Gewebes, um eine Koagulation oder auch Gewebefusion zu bewirken.

Das Maulteil bildet zusammen mit der Schneidelektrode ein Werkzeug, das an dem distalen Ende eines länglichen Schafts gehalten ist, um ein Koagulations- und Dissektionsinstrument zu bilden. Durch den Schaft hindurch erstreckt sich mindestens ein Betätigungselement, zum Beispiel in Gestalt eines Schub- und Druckelements zur Übertragung einer Betätigungsbewegung auf die beweglich gelagerte(n) Branche(n). Außerdem erstrecken sich Leitungen zur Stromversorgung von Elektroden durch den Schaft.

Typischerweise bilden der Schaft und die Werkzeugeinheit zusammen mit einem proximalen Handgriff das für den chirurgischen Eingriff vorgesehene Instrument, das von dem Instrumentenhersteller im Ganzen bereitgestellt wird.

Instrumente mit der genannten Funktionalität können auch von Operationsrobotern eingesetzt werden, die eine ferngesteuerte Operation an einem Patienten vornehmen können. Dazu müssen die Operationsroboter mit entsprechenden Instrumenten ausgerüstet werden.

Ausgehend davon ist es Aufgabe der Erfindung, eine Werkzeugeinheit bereitzustellen, die sich auf einfache Weise zur Bereitstellung eines Koagulations- und Dissektionsinstruments eignet. Insbesondere soll sich die erfindungsgemäße Werkzeugeinheit zur Bereitstellung eines von einem Operationsroboter geführten Koagulations- und Dissektionsinstruments eignen.

Die erfindungsgemäße Werkzeugeinheit umfasst eine erste Branche, die sich von einem Koppelstück weg erstreckt. Das Koppelstück ist zur Verbindung mit einem länglichen Rohrschaft eingerichtet. Beispielsweise kann es dazu einen kurzen zylindrischen Abschnitt aufweisen, der sich in einen Rohrschaft einführen und in diesem verbinden lässt. Zur Verbindung können Klebstoff, Schweißverbindungen, zum Beispiel in Form von Schweißpunkten oder Schweißnähten, Gewindeverbindungen, Niet- oder Crimpverbindungen oder dergleichen dienen. Zur Herstellung einer solchen Crimpverbindung kann beispielsweise ein Teil des distalen Randes des Rohrschafts in eine entsprechende Vertiefung des Koppelstücks eingeformt werden.

Das Koppelstück besteht vorzugsweise aus einem Metall und ist somit wiederum vorzugsweise elektrisch leitfähig. Das aus Metall bestehende Koppelstück kann mit Kunststoff ummantelt sein.

Wie das Koppelstück kann auch die erste Branche komplett aus Metall sowie alternativ ganz oder teilweise aus Kunststoff bestehen.

Gleiches gilt für eine zweite Branche der Werkzeugeinheit, die an der übrigen Werkzeugeinheit schwenkbar befestigt ist. Dazu ist ein Scharnier vorgesehen, das eine quer zu der Längsrichtung der Branche orientierte Scharnierachse aufweist. Die Längsrichtung der Branche ist von dem Koppelstück weg distal gerichtet.

Vorzugsweise sind die beiden Branchen gegeneinander elektrisch isoliert. Zur elektrischen Isolation kann durch das zwischen beiden Branchen wirksame Scharnier erbracht werden, das durch einen Kunststoffstift gebildet sein kann.

Die erfindungsgemäße Werkzeugeinheit weist an jeder der beiden Branchen jeweils wenigstens eine Koagulationselektrode sowie an einer der beiden Branchen eine Schneidelektrode und somit drei mit elektrischem Strom zu versorgende Elemente auf. Die Stromversorgung erfolgt vom proximalen Ende des Instruments her. Um dies zu ermöglichen, wird das zum Bewegen der Branche vorgesehene Betätigungselement als ein elektrischer Leiter genutzt. Zwei weitere elektrische Leiter können in Gestalt von isolierten Drähten oder Litzen in den Schaft des Instruments eingebracht sein.

Die Werkzeugeinheit wird als Baueinheit mit daran befestigtem langem oder kurzem Betätigungselement bereitgestellt.

Das kurze Betätigungselement kann in der Nähe des Koppelstücks enden, um bei Montage mit dem sich durch den Schaft erstreckenden Betätigungselement verbunden zu werden. Dies hat den Vorzug, dass der Anschluss des Betätigungselements am distalen Ende des Schafts des Instruments erfolgen kann und somit kein Ein- und Durchgriff auf den proximalen Teil des Instruments nötig ist. Außerdem kann der sich durch den Schaft erstreckende Teil des Betätigungselements an eventuell im Verlauf des Schafts vorgesehene Gelenke angepasst sein. Außerdem muss es bei Montage der Werkzeugeinheit nicht durch den Schaft und die Gelenke gefädelt werden.

Ist das Betätigungselement hingegen lang, übersteigt seine Länge die Länge des Schafts, an dem die Werkzeugeinheit zu befestigen ist. Bei der Montage der Werkzeugeinheit an dem Schaft wird das Betätigungselement durch den Schaft gefädelt.

Das sich durch den Schaft erstreckende Betätigungselement ist vorzugsweise zugfest aber biegeelastisch. So kann es sich durch Gelenke des Schafts erstrecken, ohne deren Bewegung zu behindern. Das Betätigungselement kann dann auch bis zu einem gewissen Grad Schubkräfte übertragen, um die Branchen nicht nur schließen, sondern auch öffnen zu können. Um das Betätigungselement flexibel zu gestalten, kann es beispielsweise als Federstahldraht oder Metallband, zum Beispiel Stahlband, ausgeführt sein. Es gestattet dann eine Abwinkelung beispielsweise in einem Schaftgelenk. Ist das Betätigungselement hingegen zugfest, jedoch biegeschlaff ausgeführt, kann in der Werkzeugeinheit ein Federmittel vorgesehen sein, um die Branchen federnd voneinander weg vorzuspannen, d.h. das Maulteil zu öffnen.

Auch etwaige Litzen oder sonstige Leitungen, von denen eine mit der Schneidelektrode und eine andere mit einer Versiegelungselektrode verbunden ist, können alternativ kurz oder lang ausgeführt sein. Ist die Länge der Leitungen auf ein kurzes Stück beschränkt, ist die Verbindung zu Versorgungsleitungen an dem distalen Ende des Schafts herzustellen. Die Länge der Versorgungsleitungen ist dazu so groß bemessen, dass sie soweit aus dem Schaft in distaler Richtung herausgezogen werden können, dass eine elektrische und mechanische Verbindung zu den von den Elektroden kommenden Leitungen hergestellt werden kann. Nach Herstellung der Verbindung wird die Werkzeugeinheit an das distale Schaftende heranbewegt und die Leitungen in dem Schaft untergebracht oder in proximaler Richtung nachgezogen, um die Ausbildung von Knoten und Schlingen zu vermeiden.

Es ist auch möglich, die beiden Leitungen, z.B. als Kupferlitzen oder Kupferdrähte, in einer Länge bereitzustellen, die mindestens so lang ist wie der längste Schaft, an dem die Werkzeugeinheit einzusetzen ist. In diesem Fall ist es zweckmäßig, die beiden Leitungen etwas schubsteif auszuführen. Dazu ist es zweckmäßig, sie als zweiadrige Leitung (Kabel) auszuführen. Andere Maßnahmen sind möglich, z.B. können die Leitungen aus einem elektrisch zwar weniger leitfähigen, jedoch mechanisch stabileren Material aufgebaut sei, z.B. Federstahldraht. Wenigstens einer der Leiter kann auch aus einer Kombination aus einem steifen Draht (z.B. Federstahldraht) mit einem elektrisch besser leitfähigen Draht (z.B. Kupfer oder Aluminiumdraht) bestehen. Sie können von gesonderten Isolierungen oder von einer gemeinsamen Isolierung eingehüllt sein.

Es ist zweckmäßig, das Schneidelement an der ersten Branche anzuordnen. Dies gilt insbesondere, wenn die erste Branche nicht gelenkig, sondern starr mit der übrigen Werkzeugeinheit verbunden ist. Die Stromzuführung zu der Schneidelektrode kann dann ruhend ausgeführt werden. Somit sind zwei Elektroden, nämlich die Schneidelektrode und die erste Koagulationselektrode, an der ruhenden Branche angeordnet, was die Stromzuführung über getrennte Leitungen vereinfacht und die Leitungen beim Einsatz des Instruments keinen wechselnden Biegebeanspruchungen aussetzt.

Zur Bestromung der ersten Koagulationselektrode kann eine elektrische Leitung, beispielsweise in Gestalt einer isolierten Litze oder eines isolierten Drahts direkt mit der Koagulationselektrode verbunden sein. Es ist aber auch möglich, die Branche selbst zur Stromzuleitung zu nutzen. Dies gilt unabhängig davon, ob die Branche vollständig aus Metall ausgebildet ist oder ob die Branche ein mit einem Metallinlay versehenes Kunststoffteil ist.

Die Bestromung der zweiten Branche erfolgt vorzugsweise über das Betätigungselement, das somit als Stromleiter dient. Vorzugsweise ist die zweite Elektrode die Gegenelektrode sowohl für die erste Koagulationselektrode als auch für die Schneidelektrode.

Die erfindungsgemäße Werkzeugeinheit gestattet die Ausrüstung von Operationsrobotern mit fest verbauten oder wechselbaren Instrumenten. Die Instrumente können einerseits als Rumpfinstrumente bereitgestellt werden. Sie weisen dann eine Aktoreinheit und einen am distalen Ende offenen Schaft auf. Die Aktoreinheit kann eine Betätigungseinrichtung zur mechanischen Bewegung des Werkzeugs und gegebenenfalls auch elektrische oder elektronische Mittel zur Bestromung der Elektroden aufweisen. Die Werkzeugeinheiten werden dann an das distale Ende des Schafts des Rumpfinstruments angeschlossen, wobei sowohl das Betätigungselement, wie auch die beiden zur Bestromung dienenden Leitungen angeschlossen werden. Der Anschluss kann am distalen Ende des Rumpfinstruments erfolgen. Alternativ können bei langer Ausführung des Betätigungselements und der Verbindungsmittel die mechanischen und elektrischen Verbindungen auch am proximalen Ende des Rumpfelements hergestellt werden.

Weitere Einzelheiten vorteilhafter Ausführungsformen der Erfindung ergeben sich aus Unteransprüchen sowie den Figuren der Zeichnung und der zugehörigen Beschreibung. In der Zeichnung zeigen:
Figur 1 einen Operationsroboter bei der Arbeit an einem Patienten, in schematischer Darstellung,
Figur 2 ein zu dem Operationsroboter gehöriges Instrument, in schematischer Seitenansicht,
Figur 3 das Instrument nach Figur 2, in schematischer Funktionsdarstellung,
Figur 4 bis 6 Ausführungsformen zur Bereitstellung von Instrumenten nach Figur 1 oder 2 und
Figur 7 einen proximalen Abschnitt eines Instruments zum Einsatz an einem Operationsroboter, in Prinzipdarstellung.

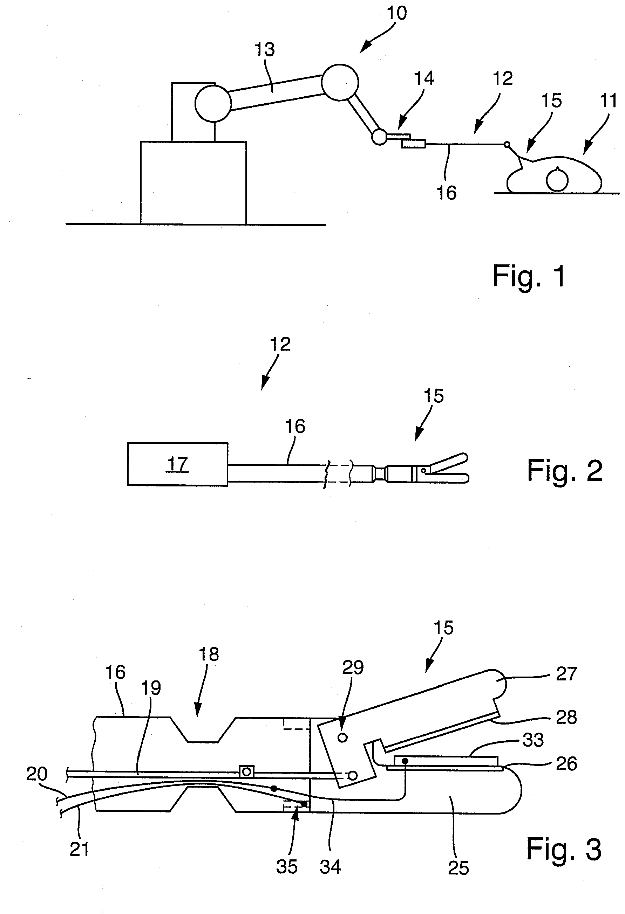

Figur 1 veranschaulicht einen Operationsroboter 10 bei der Einwirkung auf einen Patienten 11 mittels eines Instruments 12. Zu dem Operationsroboter 10 gehört ein mit einem oder mehreren Gelenken versehener Arm 13, der an seinem äußeren Ende 14 das Instrument 12 trägt. Der Arm 13 führt das Instrument 12, um mit diesem an dem Patienten Operationen durchzuführen. Der Operationsroboter kann manuell oder auch maschinell geführt sein.

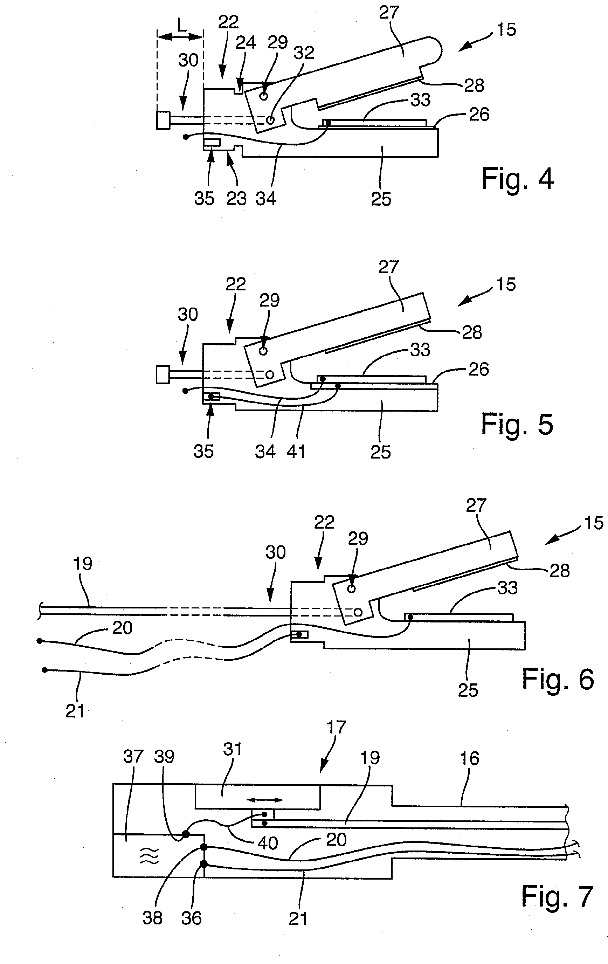

Das Instrument 12 ist in Figur 2 gesondert veranschaulicht. Es handelt sich um ein Koagulations- und Dissektionsinstrument, zu dem eine Werkzeugeinheit 15 gehört, die an dem distalen Ende eines länglichen Schafts 16 angeordnet ist. An dem proximalen Ende des Schafts 16 ist eine Aktoreinheit 17 vorgesehen, die gesondert in Figur 7 veranschaulicht ist und die zur Bewegung und zum Betrieb der Werkzeugeinheit 15 dient.

Die Werkzeugeinheit 15 ist, wie die Figuren 2 und 3 erkennen lassen, mit dem Schaft 16 verbunden, der in der Nähe seines distalen Endes ein Gelenk 18 aufweisen kann. Dieses Gelenk 18 ist typischerweise darauf ausgelegt, um wenigstens eine Achse schwenken zu können. Zur Bewirkung der Schwenkbewegung können in der Aktoreinheit 17 nicht weiter veranschaulichte Aktoren vorgesehen sein, die beispielsweise über ebenfalls nicht veranschaulichte Zugmittel ein Abwinkeln des Schafts 16 an dem Gelenk 18 bewirken.

Durch den Schaft 16 und das Gelenk 18 erstrecken sich Mittel zur Betätigung der Werkzeugeinheit 15, nämlich mindestens ein Betätigungselement 19, sowie mindestens zwei elektrische Leitungen 20, 21, beispielsweise in Gestalt isolierter Litzen oder Drähte. Das Betätigungselement 19 kann zum Beispiel als flexibles Metallband ausgebildet sein, das Zugkräfte und, zumindest im beschränkten Maße, auch Druckkräfte übertragen kann. Es ist dabei flexibel ausgebildet, um sich an eine Biegung in dem Bereich des Gelenks 18 anpassen zu können.

Die Werkzeugeinheit 15 der in Figur 3 veranschaulichten Ausführungsform ist in Figur 4 gesondert veranschaulicht. Sie weist ein Koppelstück 22 mit einem zylindrischen Ansatz 23 auf, der dazu eingerichtet ist, mit dem distalen Ende des Schafts 16 fest verbunden zu werden. Dazu kann der zylinderförmige Ansatz 23 in den Schaft 16 einschiebbar sein und zur formschlüssigen Befestigung des Schafts 16 an dem Koppelstück 22 Formschlussstrukturen 24 aufweisen. Eine solche Formschlussstruktur 24 kann beispielsweise eine sich um den Ansatz 23 herum erstreckende Nut, eine einzelne Vertiefung oder eine Tasche oder Öffnung sein, in die ein mit dem Schaft zusammenwirkendes Befestigungselement einsetzbar ist. Die Verbindung zwischen der Werkzeugeinheit 15 und dem Schaft 16 kann unlösbar und somit dauerhafter Natur sein. Es ist auch möglich, zwischen dem Schaft und der Werkzeugeinheit eine lösbare Verbindungseinrichtung vorzusehen, wie z.B. eine Gewindeverbindung, eine Steckverbindung oder eine Rastverbindung.

Das Koppelstück 22 trägt eine erste Branche 25, die vorzugsweise starr mit dem Koppelstück 22 verbunden ist und sich in distaler Richtung von dem Koppelstück 22 weg erstreckt. Alternativ kann die erste Branche auch beweglich angeordnet sein. Das Koppelstück 22 und die erste Branche 25 können aus einem einzigen Teil beispielsweise aus Metall oder auch aus Kunststoff bestehen. Jedenfalls ist an der ersten Branche 25 eine erste Koagulationselektrode 26 befestigt. Wenn die erste Branche 25 und das Koppelstück 22 aus Metall bestehen, können die Branche 25 und das Koppelstück 22 als elektrischer Leiter und somit als elektrisches Verbindungsmittel zur Verbindung der Koagulationselektrode 26 mit der Leitung 21 dienen. Wenn das Koppelstück 22 und die erste Branche 25 aus Kunststoff bestehen und ein Metallinlay enthalten, kann das Metallinlay als elektrischer Leiter zur Verbindung der Koagulationselektrode 26 mit der Leitung 21 dienen. Alternativ kann die Koagulationselektrode 26 über ein Kabel oder einen sonstigen Leiter (Draht) kontaktiert sein. Die Branche oder das Inlay sind dann potentialfrei.

An dem aus dem Koppelstück 22 und der ersten Branche 25 bestehenden Bauteil ist eine zweite Branche 27 schwenkbar gelagert, die eine zweite Koagulationselektrode 28 trägt. Die zweite Branche 27 ist bei einem Scharnier 29 schwenkbar gelagert, das eine quer zu dem Schaft 16 orientierte Scharnierachse festlegt. Wenn die Branchen 25, 27 aus Metall bestehen, ist das Scharnier 29 elektrisch isolierend ausgebildet. Beispielsweise weist das Scharnier 29 dann einen aus Kunststoff bestehenden oder mit Kunststoff ummantelten Scharnierstift auf, um den die zweite Branche 27 schwenken kann. Alternativ kann das Scharnier auch aus mit einem isolierenden Überzug versehenen, ineinandergreifenden Metallteilen oder aus mehreren Kunststoffteilen z.B. nach dem Vorbild der EP 3 632 354 B1 bestehen.

Die zweite Branche 27 kann vollständig aus Metall bestehen und somit eine elektrische Verbindung zwischen der Koagulationselektrode 28 und einem kurzen Betätigungselement 30 herstellen, das an seinem distalen Ende mit der Branche 27 und mit dem sich durch den Schaft 16 erstreckenden Betätigungselement 19 verbunden ist. Das Betätigungselement 19 ist an seinem proximalen Ende mit einem Linearaktor 31 (Figur 7) verbunden. Das kurze Betätigungselement 30 kann als biegesteife Metallstange ausgelegt sein und nach außen isoliert sein, z.B. durch eine Beschichtung oder einen Schrumpfschlauch.

Die zweite Branche 27 kann alternativ aus einem Kunststoffteil bestehen, das ein Metallinlay aufweist. Das Metallinlay erstreckt sich dann von der zweiten Koagulationselektrode 28 bis zu dem Anlenkpunkt 32, bei dem das Betätigungselement 30 mit der Branche 27 verbunden ist. Anstelle des Anlenkpunktes 32 kann auch eine Kulisse vorgesehen sein, so dass zwischen dem Betätigungselement 30 der Branche 27 ein Kulissengetriebe gebildet ist.

An der ersten, feststehenden Branche 25 ist eine Schneidelektrode 33 angeordnet, die beispielsweise in einer Längsausnehmung der ersten Koagulationselektrode 36 platziert sein kann. Die Schneidelektrode 33 und die erste Koagulationselektrode 26 sind somit voneinander elektrisch isoliert. Vorzugsweise ist die Schneidelektrode 33 starr an der Branche 25 angeordnet. Sie kann alternativ federnd gelagert sein. Vorzugsweise ist sie aber jedenfalls in Längsrichtung (d.h. nach distal und proximal) fest angeordnet. Dies hat den Vorteil, dass die bewegliche Branche nur eine einzige elektrische Zuleitung aufweist, die durch das ohnehin vorhandene Betätigungselement 30 gebildet sein kann. In einer alternativen Ausführungsform ist die Schneidelektrode in der beweglichen Branche 27 fest oder federnd angeordnet. Das Widerlager ist dann in der feststehenden Branche 25 angeordnet.

Die gegenüberliegende zweite Koagulationselektrode 26 weist im Auftreffbereich der Schneidelektrode 33 eine schlitzartige Ausnehmung auf, in der vorzugsweise ein flexibles Widerlage zum Beispiel in Gestalt eines Gummi- oder Silikonelements angeordnet ist. Wenn ein nicht nachgiebiges Widerlager vorgesehen ist, wird es bevorzugt, die Schneidelektrode nachgiebig zu lagern.

Die Schneidelektrode 33 ist mit einer elektrischen Anschlussleitung 34 verbunden. Diese erstreckt sich in proximaler Richtung so weit, dass sie aus dem Koppelstück 22 etwas herausragt. Ebenso ragt das kurze Betätigungselement 30 um die Länge L etwas aus dem Koppelstück 22 heraus. Vorzugsweise sind die Längen, mit der die Anschlussleitung 34 und das Betätigungselement 30 aus dem Koppelstück 22 herausragen geringer, vorzugsweise deutlich geringer, als die Länge der Branche 25 in distaler Richtung. Alternativ kann die Anschlussleitung 34 länger als der gesamte Schaft 16 sein.

An dem Koppelstück 22 kann außerdem eine Anschlusseinrichtung 35 für die Leitung 21 vorgesehen sein. Die Anschlusseinrichtung 35 kann beispielsweise eine Klemmeinrichtung sein, in der die Leitung 21 gegen das Koppelstück 22 festgeklemmt wird. Die Anschlusseinrichtung 35 kann auch eine an dem Koppelstück 22 ausgebildete Biegelasche zum Festklemmen des Endes der Leitung 21, ein Schneidklemmschlitz, oder eine Ausnehmung sein, in der das abisolierte Ende der Leitung 21 festgeschweißt oder festgelötet werden kann.

Damit wird die elektrische Verbindung zwischen der ersten Koagulationselektrode 26 und einem entsprechenden Anschluss 36 eines Generators 37 hergestellt, der in der Aktoreinheit 17 angeordnet sein kann. Des Weiteren wird bei der Befestigung der Werkzeugeinheit 15 an dem Schaft 16 eine Verbindung zwischen der Leitung 34 und der Leitung 20 hergestellt, die sich wie die Leitung 21 durch den gesamten Schaft 16 bis in die Aktoreinheit 17 erstreckt. Die Leitung 20 ist an einen weiteren Anschluss 38 des Generators 37 angeschlossen, um von dem Generator 37 mit Schneidspannung versorgt zu werden. Ein dritter Anschluss 39 des Generators ist über eine geeignete elektrische Kontaktierung, beispielsweise in Gestalt einer flexiblen Leitung 40, mit dem proximalen Ende des Betätigungselements 19 verbunden. Auf diese Weise wird die zweite Koagulationselektrode 28 von dem Anschluss 39 über das Betätigungselement 19/30 mit Strom versorgt.

Der Generator 37 ist dazu eingerichtet, zwischen den Anschlüssen 36 und 39 eine Koagulationsspannung abzugeben. Diese ist eine hochfrequente Hochspannung mit einer Frequenz oberhalb 100 kHz, typischerweise im Bereich von 200 kHz bis 1 MHz. Die Spannung (Spitze zu Spitze) liegt typischerweise im Bereich von mehr als 100 V, jedoch weniger als 300 V. Der Generator 37 ist außerdem drauf eingerichtet, zwischen dem Anschluss 38 und dem Anschluss 39 eine Schneidspannung abzugeben. Diese ist größer als die Koagulationsspannung, weist jedoch vorzugsweise die gleiche Frequenz auf, wie diese. Die Schneidspannung und die Koagulationsspannung können gleichzeitig oder zeitversetzt an den jeweiligen Anschlüssen und Elektroden anliegen.

Zur Bereitstellung des Instruments 12 wird wie folgt vorgegangen:
Zunächst wird ein Rumpfinstrument bereitgestellt, das die Aktoreinheit 17 und den Schaft 16 umfasst. Bei dieser Ausführungsform sind sowohl die Leitungen 20, 21, als auch das lange Betätigungselement 19 bereits in den Schaft 16 eingezogen. Es wird nun die Werkzeugeinheit 15 nach Figur 4 an das distale Ende des Schafts 16 gebracht und die Leitung 20 mit der Anschlussleitung 34 verbunden, sowie die Leitung 21 an die Anschlusseinrichtung 35 angeschlossen. Außerdem wird das kurze Betätigungselement 30 mit dem langen Betätigungselement 19 verbunden, das ein Stück weit aus dem Koppelstück 22 herausragt oder zumindest in eine solche Position gebracht werden kann. Die Verbindung kann mit einem Niet durch eine Crimpverbindung, durch einen Schweißpunkt oder durch ähnliche geeignete Maßnahmen erfolgen. Gleiches gilt für die Verbindung zwischen der Anschlussleitung 34 und der Leitung 20. Sind diese Verbindungen hergestellt, wird das Koppelstück 22 in das distale Ende des Schafts 16 eingesetzt oder an dieses angesetzt und mit dem Schaft verbunden. Die Verbindung kann auf jede geeignete Weise, beispielsweise formschlüssig oder durch stoffschlüssige Verbindung beispielsweise Schweißen, Kleben oder dergleichen erfolgen.

Figur 5 zeigt eine abgewandelte Ausführungsform der Werkzeugeinheit 15, bei der das Koppelstück 22 und die Branche 25 aus elektrisch isolierendem Material, beispielsweise Kunststoff, gefertigt sind. Deswegen ist zur Kontaktierung der ersten Koagulationselektrode 26 eine Anschlussleitung 41 vorgesehen, die sich wie die Anschlussleitung 34 zumindest bis zu der Anschlusseinrichtung 35 oder auch darüber hinaus erstreckt. Ein Vorteil dieser Ausführungsform liegt in der zuverlässigen und einfachen Isolation der Branche 25 gegenüber dem Betätigungselement 30. Im Übrigen und ansonsten gilt die im Zusammenhang mit der Werkzeugeinheit 15 nach Figur 4 gegebene Beschreibung entsprechend.

Eine weiter abgewandelte Form der Werkzeugeinheit 15 veranschaulicht Figur 6. Diese zeichnet sich durch eine Minimierung der Anzahl der nötigen Verbindungsstellen aus. Von dem Koppelstück 22 erstrecken sich sowohl die Leitungen 20, 21, als auch das Betätigungselement 19/30 jeweils nahtlos in proximaler Richtung über eine Länge, die die Länge des längsten anzunehmenden Schachts 16 übersteigt. In dieser Ausführungsform kann die Werkzeugeinheit 15 an Kunden geliefert werden, die aus anderer Quelle ein Rumpfinstrument gemäß Figur 7 beziehen. Zur Komplettierung des Instruments werden dann das Betätigungselement 19 und die Leitungen 20 durch den Schaft 16 hindurch bis in die Aktoreinheit 17 geführt und dort angeschlossen. Gegebenenfalls können das Betätigungselement 19 und die Leitungen 20, 21 auf das benötigte Maß eingekürzt werden. Es werden dann das Betätigungselement 19 an den Abtrieb des Linearaktuators 31 und die Leitungen 20, 21 an die Anschlüsse 38, 36 des Generators 37 angeschlossen. Außerdem wird das Koppelstück 22 mit dem distalen Ende des Schafts 16 verbunden. Sowohl die Leitungen 20, 21, als auch das Betätigungselement 19 sind biegeflexibel oder abschnittsweise biegeflexibel. Sollte der Schaft 16 das Gelenk 18 oder gegebenenfalls auch mehrere Gelenke aufweisen, widersetzen sie sich nicht einer Biegung im Gelenkbereich.

Eine erfindungsgemäße Werkzeugeinheit 15 weist zwei gegeneinander elektrisch isolierte oder aus isolierendem Material bestehende Branchen 25, 27 auf, an denen Koagulationselektroden 26, 28 vorgesehen sind. Während die erste Branche 25 an der Werkzeugeinheit starr gehalten ist, ist die zweite Branche 27 schwenkbar. Um der zweiten Branche 27 eine Schwenkbewegung zu erteilen, ist ein Betätigungselement 30 in Form einer Zug-und-Druckstange, beispielsweise in Gestalt eines flexiblen Blechstreifens vorgesehen, der zugleich als elektrischer Leiter dient, um der zweiten Koagulationselektrode 28 Strom zuzuführen. Die erste Koagulationselektrode 26 ist über ein Anschlussmittel mit einer Zuleitungslitze 21 verbunden. Das Anschlussmittel kann die Branche 25 selbst, ein in dieser vorgesehenes elektrisch leitendes Inlay oder eine Anschlussleitung 41 sein. Eine zweite Anschlussleitung 34 dient zur Kontaktierung der Schneidelektrode 33, das heißt zur Stromzuführung zu derselben. Bei einer abgewandelten Variante ist das Betätigungselement 30 im starren Bereich des Robotikarms oder des Schafts 16 eine Stange und im Gelenkbereich 18 des Arms oder Schafts 16 ein Kabelzug. Dieser Kabelzug ist dann einigermaßen flexibel und bis zu gewissem Maß auch zur Übertragung von Schubkräften geeignet.

In einer ersten Ausführungsform sind das Betätigungselement 30 und die Anschlussleitung 34 kurz. Der Koagulationsstrom wird über die Leitung 21 und das Anschlussmittel 35 zugeführt, während der Schneidstrom über die kurze Anschlussleitung 34 zugeführt wird. Bei einer zweiten Ausführungsform sind das Betätigungselement 19 sowie die Leitungen 20, 21 lang ausgeführt, so dass sie durch den gesamten Schaft des Instruments geführt und an dem proximalen Ende des Schafts in der Aktoreinheit 17 mit einem speisenden Generator 37 verbunden werden können.

Die erfindungsgemäße Werkzeugeinheit 15 gestattet die Nutzung durch verschiedene Hersteller von Rumpfinstrumenten und/oder Operationsrobotern. Eine Anpassung an spezielle Gegebenheiten ist beispielsweise durch Kürzung des Betätigungselements 19 sowie der Leitungen 20, 21 möglich. Bei der bevorzugten Ausführungsform wird das Maulteil mit langen daran befestigten Kabeln für die Schneidelektrode 33 und die untere Versiegelungselektrode 26 geliefert, die über das Koppelstück 22 kontaktiert wird. Der Robotikhersteller stellt das potentialführende Zug-Schubelement 30 zur Verfügung und befestigt es direkt in der Drehkulisse oder an dem Anlenkpunkt 32 der oberen Branche 27.

### Bezugszeichen:

- 10: Operationsroboter
- 11: Patient
- 12: Instrument
- 13: Arm
- 14: äußeres Ende des Arms 13
- 15: Werkzeugeinheit
- 16: Schaft
- 17: Aktoreinheit
- 18: Gelenk
- 19: langes Betätigungselement
- 20: Leitung für Schneidelektrode
- 21: Leitung für Koagulationselektrode
- 22: Koppelstück
- 23: Ansatz
- 24: Formschlussstruktur
- 25: erste Branche
- 26: erste Koagulationselektrode
- 27: zweite Branche
- 28: zweite Koagulationselektrode
- 29: Scharnier
- 30: kurzes Betätigungselement
- 31: Linearaktor
- 32: Anlenkpunkt
- 33: Schneidelektrode
- 34: Anschlussleitung
- 35: Anschlusseinrichtung
- 36: Anschluss
- 37: Generator
- 38: Anschluss
- 39: Anschluss
- 40: Kontaktmittel
- 41: Anschlussleitung

## Patentansprüche

1. Werkzeugeinheit (15) insbesondere für einen Operationsroboter (10),
mit einem Koppelstück (22), das zur Verbindung einem Operationsroboter (10) eingerichtet ist,
mit einer ersten, sich von dem Koppelstück (22) weg erstreckenden Branche (25),
mit einem an dem Koppelstück (22) und einer zweiten Branche (27) vorgesehenen elektrisch isolierendem Scharnier (29), durch das die zweite Branche (27) auf die erste Branche (25) hin und von dieser weg um eine Schwenkachse schwenkbar gelagert ist,
mit einem Betätigungselement (19, 30), das in einem Abstand zu der Schwenkachse mit der zweiten Branche (27) schwenkbar und elektrisch leitend in Berührung oder Verbindung stehend angeordnet ist,
mit einer isolierten elektrischen Schneidstromleitung (20), die mit der Schneidelektrode (33) verbunden ist und die sich wenigstens bis in das Koppelstück erstreckt, und
mit einem elektrischen Verbindungsmittel (25, 35, 41), das zur elektrischen Verbindung zwischen einer isolierten elektrischen Koagulationsstromleitung (21) und der ersten Elektrode (26) eingerichtet ist.

2. Werkzeugeinheit nach Anspruch 1, **dadurch gekennzeichnet, dass** die Schneidelektrode (33) an der ersten Branche (25) angeordnet ist.

3. Werkzeugeinheit nach einem der vorstehenden Ansprüche, **dadurch gekennzeichnet, dass** die erste Branche (25) mit dem Koppelstück (22) starr verbunden ist.

4. Werkzeugeinheit nach einem der vorstehenden Ansprüche, **dadurch gekennzeichnet, dass** die erste Branche (25) eine erste Koagulationselektrode (26) und die zweite Branche (27) eine zweite Koagulationselektrode (28) aufweist.

5. Werkzeugeinheit nach einem der vorstehenden Ansprüche, **dadurch gekennzeichnet, dass** die erste Koagulationselektrode (26) mit der ersten Branche (25) elektrisch verbunden ist, wobei die erste Branche (25) das elektrische Verbindungsmittel ist, an das die Koagulationsstromleitung (21) über eine Anschlusseinrichtung (35) anschließbar ist.

6. Werkzeugeinheit nach einem der vorstehenden Ansprüche, **dadurch gekennzeichnet, dass** die erste Koagulationselektrode (26) von der ersten Branche (25) elektrisch isoliert ist, wobei das Verbindungsmittel eine sich von der ersten Koagulationselektrode (26) zu dem Koppelstück erstreckende Verbindungsleitung (41) ist.

7. Werkzeugeinheit nach einem der vorstehenden Ansprüche, **dadurch gekennzeichnet, dass** das Koppelstück (22) zur Verbindung mit einem distalen Ende eines Robotergelenks (18) oder robotisch bewegbaren Schafts (16) eingerichtet ist.

8. Werkzeugeinheit nach einem der vorstehenden Ansprüche, **dadurch gekennzeichnet, dass** das Betätigungselement (19, 30) sich in proximaler Richtung über das Koppelstück (22) hinaus erstreckend ausgebildet ist.

9. Werkzeugeinheit nach einem der vorstehenden Ansprüche, **dadurch gekennzeichnet, dass** die Schneidstromleitung (20) sich in proximaler Richtung über das Koppelstück (22) hinaus erstreckend ausgebildet ist.

10. Werkzeugeinheit nach einem der vorstehenden Ansprüche, **dadurch gekennzeichnet, dass** die Koagulationsstromleitung (21) sich in proximaler Richtung über das Koppelstück (22) hinaus erstreckend ausgebildet ist.

11. Werkzeugeinheit nach einem der vorstehenden Ansprüche, **dadurch gekennzeichnet, dass** das Betätigungselement (19, 30) und/oder die Schneidstromleitung (20) und/oder die Koagulationsstromleitung (21) sich in proximaler Richtung über das Koppelstück (22) über ein Vielfaches der Länge der Werkzeugeinheit (15) hinaus erstreckend ausgebildet ist.

12. Werkzeugeinheit nach einem der vorstehenden Ansprüche, **dadurch gekennzeichnet, dass** das Koppelstück (22) mit einer Anschlussstruktur (24) für ein distales Ende eines Robotergelenks (18) oder robotisch bewegbaren Schafts (16) versehen ist.

13. Werkzeugeinheit nach einem der vorstehenden Ansprüche, **dadurch gekennzeichnet, dass** das Betätigungselement (19, 30) und/oder die Schneidstromleitung (20) und/oder die Koagulationsstromleitung (21) sich in proximaler Richtung mit höchstens einer Länge (L) aus dem Koppelstück (22) herausragend ausgebildet ist, die geringer ist, als die Länge der Werkzeugeinheit (15) in distaler Richtung.

14. Werkzeugeinheit nach einem der vorstehenden Ansprüche, **dadurch gekennzeichnet, dass** das Betätigungselement (19, 30) und/oder die Schneidstromleitung (20) und/oder die Koagulationsstromleitung (21) eine Anschlusseinrichtung aufweist.

15. Verfahren zur Befestigung eines Werkzeugs (12) an einem Operationsroboter, wobei bei dem Verfahren das einem der Ansprüche 1 bis 12 entsprechende Werkzeug (12) an ein distales Ende eines Schafts (16) eines Operationsroboters (10) heran geführt wird, wonach das Betätigungselement (19, 30), die Koagulationsstromleitung (21) und die Schneidstromleitung (20) durch den Schaft geführt werden, wonach das Betätigungselement (19, 30) mechanisch an einen Aktor (31) des Operationsroboters (10) und elektrisch an eine elektrische Versorgungseinrichtung (37) angeschlossen wird, wobei die Koagulationsstromleitung (21) sowie die Schneidstromleitung (20)an ihren distalen Enden ebenfalls an die elektrische Versorgungseinrichtung (37) angeschlossen werden.
